# EUROPEAN PATENT APPLICATION

(11) **EP 1 600 106 A1**
(43) Date of publication of application: **30.11.2005**
(21) Application number: 04715452.1
(22) Date of filing: 27.02.2004
(51) Int. Cl.: A61B 8/08

(54) **ULTRASONOGRAPHIC DISPLAY DEVICE**

(30) Priority: 28.02.2003 JP 2003054568
(71) Applicant: MATSUSHITA ELECTRIC INDUSTRIAL CO., LTD., Kadoma-shi, Osaka 571-8501 (JP)
(72) Inventor: NISHIGAKI, Morio, (JP); HAGIWARA, Hisashi, (JP)
(74) Representative: Schorr, Frank Jürgen
(86) International application number: PCT/JP2004/002352
(87) International publication number: WO 2004/075755

(57) **Abstract**

There is provided an ultrasonic image displaying system capable of displaying an image of a fetus that is easy for a subject to understand without requiring a complicated operation by an operator. The ultrasonic image displaying system is provided with a display unit 104 for displaying an image of a fetus, a part data recording part 106 for recording part images, and a synthesis unit 103 for adding a part image read from the part data recording part 106 to a region designated by the operator on the image of the fetus displayed on the display unit 104.

## Description

### Technical Field

The present invention relates to an ultrasonic image displaying system for displaying an image created based on information obtained from the inside of a body by transmitting and receiving ultrasonic waves.

### Background Art

The principle of ultrasonic image displaying systems already has been known publicly. That is, two-dimensional or three-dimensional information of the inside of a body is obtained by transmitting and receiving ultrasonic waves repeatedly to/from the inside of the body by using a pulse generator, and an image created based on the obtained information is displayed.

Ultrasonic image displaying systems are used in a wide range of fields, and in obstetrics it is becoming popular to provide an ultrasonic image of a fetus as a service to clients as shown in the article of "Obstetric Services" in the Yomiuri Shimbun on July 30, 2002, for example.

However, although screen displays of ultrasonic image displaying systems have become easy to see as compared with conventional ones, they are intended for experts, and thus it is still difficult in many cases for a person who is not familiar with these displays to determine what is displayed.

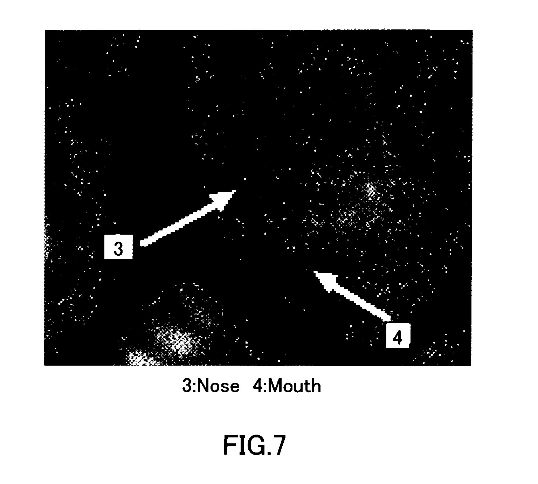

Therefore, in many cases, an operator writes names of regions such as a nose and a mouth on an image as shown in Figure 7, or provides a verbal explanation while viewing a screen of an ultrasonic image displaying system.

The following patent document (JP 8(1996)-206117 A) describes conventional ultrasonic diagnostic equipment that displays a contour image of a fetus.

However, a conventional method in which a verbal explanation is provided or characters are written has a problem that patients do not quite understand what is displayed. Further, conventionally, an operator provides an explanation by drawing a picture based on an image displayed by an ultrasonic image displaying system. However, in such a case, there is a problem that if the operator is not used to drawing a picture or the like, the picture provides an image that is not actually true, or it takes time to draw the picture.

### Disclosure of Invention

It is an object of the present invention to solve the above-mentioned problems and to provide an ultrasonic image displaying system that is less burdensome for an operator and displays an image that is easy for a patient to understand.

In order to achieve the above-mentioned object, an ultrasonic image displaying system according to the present invention includes: a display unit for displaying an image of a fetus; a part image recording unit for recording part images; and an image synthesis unit for reading a part image corresponding to a region designated by an operator from the part image recording unit, and superimposing the read part image on the image displayed by the display unit.

As described above, since the part images for eyes, a nose, a mouth, and the like prepared in advance are superimposed on the displayed image, it is possible to display easily the part images that aid a subject's understanding only by designating a position of a region.

It is preferable that in the ultrasonic image displaying system according to the present invention, the part image recording unit records as the part images a plurality of types of part images with respect to at least one facial region. It is preferable that the system further includes a selection operation unit for allowing the operator to select any of the plurality of types of part images recorded by the part image recording unit, wherein the part image selected by the selection operation unit is superimposed on the image.

With this configuration, by selecting an appropriate part image from the plurality of types of part images prepared in advance, a variety of descriptive images can be displayed easily.

It is preferable that the ultrasonic image displaying system according to the present invention further includes a correction unit for correcting a turning angle of the part image in the display unit. Therefore, it is possible to correct a display state by turning the part image in accordance with a direction and a tilt of a face of a fetus, whereby more natural descriptive images can be displayed.

It is preferable that the ultrasonic image displaying system according to the present invention having any one of the above-mentioned configurations includes an output unit for outputting the image of the fetus and the part image added to the image in at least one form of printing, analog data output, and digital data output. This allows a subject to take home the image of the fetus to which the part images are added so as to show it to her family and the like and store it.

It is preferable that the ultrasonic image displaying system according to the present invention includes a part image display unit for separating the plurality of part images superimposed by the image synthesis unit from the image of the fetus while preserving a relative positional relationship of the part images, and displaying the separated part images in a region different from a display region for displaying the image of the fetus. With this configuration, since the part images are displayed in a region different from a display region for displaying the image of the fetus, descriptive images that are easy for a subject to understand can be displayed without spoiling an original image (image of the fetus). Further, since the part images are superimposed on the original image initially and then separated from the original image while a relative positional relationship between the part images is preserved, the part images that are true to the original image can be obtained.

Further, it is also preferable that this configuration includes an output unit for outputting the image of the fetus and the part images displayed by the part image display unit as separate images, wherein the output unit performs output in at least one form of printing, analog data output, and digital data output.

### Brief Description of Drawings

Figures 1A to 1D are image pictures showing a manner in which descriptive images are added to an image of a fetus displayed on a screen in an ultrasonic image displaying system according to a first embodiment of the present invention.
Figure 2 is an exemplary block diagram of the ultrasonic image displaying system according to the first embodiment of the present invention.
Figures 3A to 3E are image pictures showing a manner in which descriptive images are added to an image of a fetus displayed on a screen in an ultrasonic image displaying system according to a second embodiment of the present invention.
Figure 4 is an exemplary block diagram of the ultrasonic image displaying system according to the second embodiment of the present invention.
Figure 5A and 5B, respectively, are an image picture obtained as a result of adding descriptive images to an image of a fetus displayed on a screen and a view showing the original image and the descriptive images displayed separately in an ultrasonic image displaying system according to a third embodiment of the present invention.
Figure 6 is an exemplary block diagram of the ultrasonic image displaying system according to the third embodiment of the present invention.
Figure 7 is an exemplary image picture used to describe an image of a fetus according to a conventional example of the present invention.

### Description of the Invention

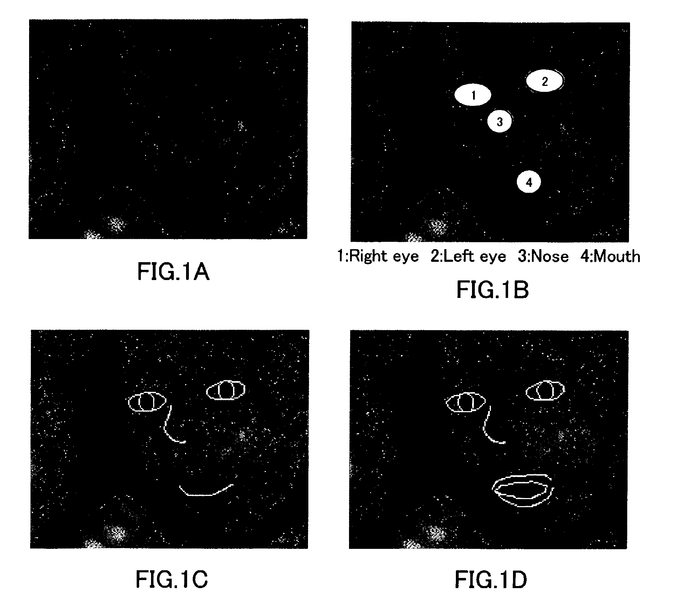

Hereinafter, embodiments of the present invention will be described with reference to Figures 1 to 6.

### (First Embodiment)

Figures 1A to 1D show an example of a manner in which descriptive images are added to an ultrasonic diagnostic image in an ultrasonic image displaying system according to a first embodiment of the present invention.

Figure 1A is an ultrasonic diagnostic image (original image). On this image, an operator designates a position of a part such as an eye, a nose, and a mouth as shown in Figure 1B using a mouse, a trackball, or the like. For example, when the operator designates positions of eyes, with respect to each of the left eye and the right eye, the operator selects a position (e.g., central position) on the ultrasonic diagnostic image as well as selects a part type (left eye or right eye) corresponding to the selected position. The ultrasonic image displaying system displays a part image prepared in advance in the corresponding portion as shown in Figure 1C based on the designated position information and part type. A plurality of types of part images are prepared with respect to each region, which can be selected by the operator. For example, since the mouth is open in the original image in Figure 1A, a part image for the mouth can be changed to an image of an open mouth as shown in Figure 1D.

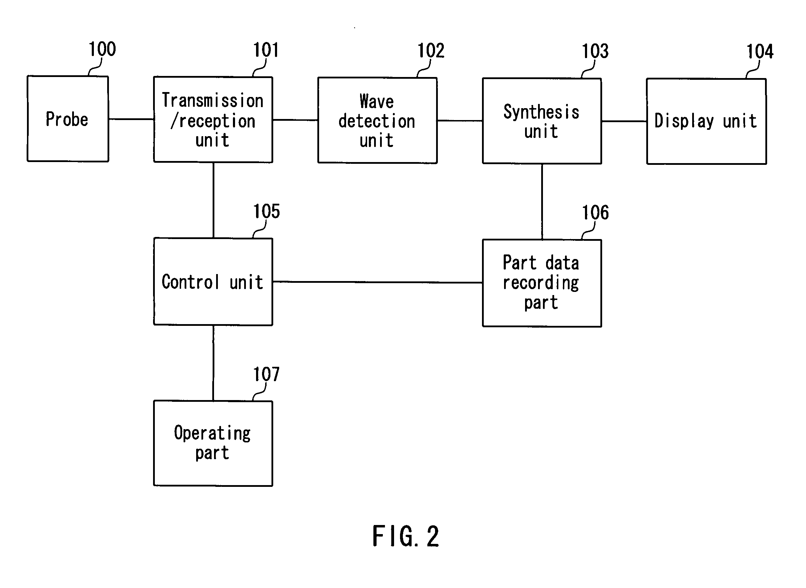

Figure 2 shows an exemplary configuration of the ultrasonic image displaying system according to the present embodiment for producing the displays as shown in Figures 1A to 1D.

As shown in Figure 2, the ultrasonic image displaying system of the present embodiment is provided with a probe 100, a transmission/reception unit 101, a wave detection unit 102, a synthesis unit 103, a display unit 104, a control unit 105, a part data recording part 106, and an operating part 107.

The thus configured ultrasonic image displaying system of the present embodiment is operated in the following manner under the control of the control unit 105.

An electrical pulse generated by the transmission/reception unit 101 is converted into an ultrasonic signal by the probe 100 and irradiated to the inside of a body not shown. A signal reflected from the inside of the body is received by the transmission/reception unit 101, detected by the wave detection unit 102, and then input to the synthesis unit 103.

The synthesis unit 103, which has a scan conversion function, creates an image (ultrasonic diagnostic image) of an object inside the body and transmits the created image to the display unit 104, where it is displayed. Further, the synthesis unit 103 also has a function of synthesizing a part image extracted from the part data recording part 106 and the ultrasonic diagnostic image. When the operator designates a position of a part and a part type on the ultrasonic diagnostic image displayed on the display unit 104 by operating the operating part 107, data of a part image recorded in the part data recording part 106 are called with respect to the designated part type, the called part image and the ultrasonic diagnostic image are synthesized by the synthesis unit 103, and the synthesized image is displayed on the display unit 104.

Specifically, the synthesis unit 103 obtains coordinates of the position designated by the operator, and superimposes the data of the part image called from the part data recording part 106 on data of the coordinates on the ultrasonic diagnostic image. The superimposition of the part image on the ultrasonic diagnostic image may be performed in either of the following manners (1) and (2) or can be performed by another arbitrary image processing method. That is, (1) the data of the part image cover the image data in the corresponding region of the ultrasonic diagnostic image completely, or (2) the data of the part image are superimposed on the image data in the corresponding region of the ultrasonic diagnostic image in a state where the image data are visible to some extent.

### (Second Embodiment)

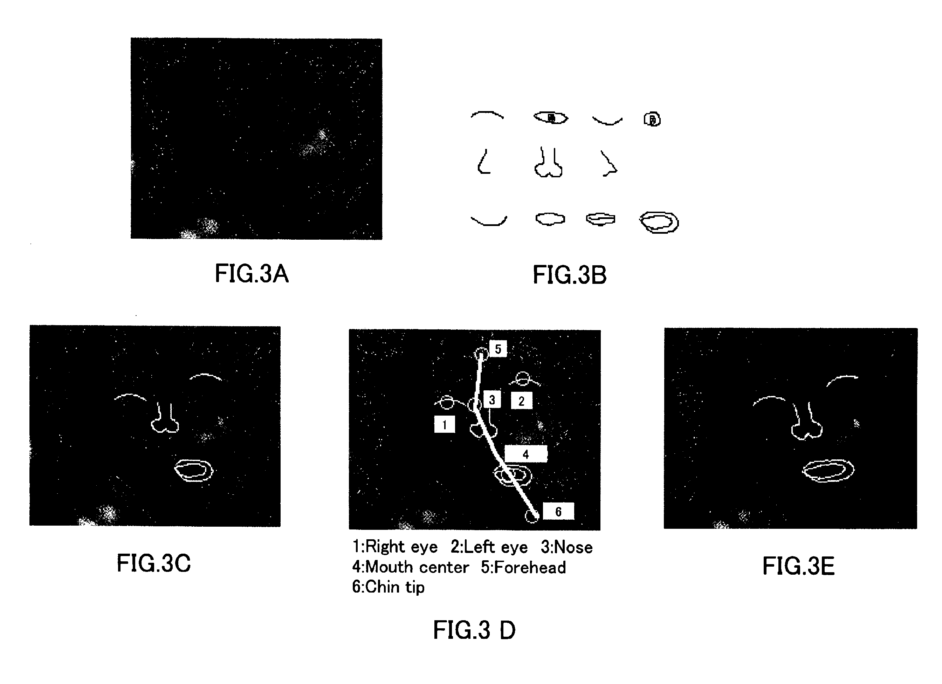

Figures 3A to 3E show an example of a manner in which descriptive images are added to an ultrasonic diagnostic image in an ultrasonic image displaying system according to a second embodiment of the present invention.

Figure 3A is an ultrasonic diagnostic image (original image). An operator selects a desirable part image as appropriate from part images prepared in advance with respect to respective regions as shown in Figure 3B, and superimposes the selected part image on the original image. The displays in Figures 3A and 3B may be provided on a divided screen of the ultrasonic image displaying system or on two display screens prepared respectively. Further, there is no need to display all the parts at the same time. For example, it is also possible that only part images for a mouth are displayed initially and then part images for a nose are displayed after a part image for the mouth is superimposed on the ultrasonic diagnostic image. It is preferable that the operation of superimposing the part image on the ultrasonic diagnostic image is performed in a drag-and-drop manner with a mouse or using a trackball, which makes the operation easy.

Figure 3C shows an example of a state where the part images are superimposed on the ultrasonic diagnostic image, in which a face on the original image is tilted slightly. The ultrasonic image displaying system of the present embodiment allows the operator to select each of the part images and modify a position and a turning angle thereof. Alternatively, the position and the turning angle of each of the part images can be corrected by designating positions of a forehead-a nose-a mouth center-a chin tip as well as eyes, respectively, as shown in Figure 3D.

For example, in order to correct the display angles of the part images for the eyes, an angle of a line connecting the positions of the respective eyes designated by the operator with respect to a horizontal line is calculated, and the part images for the eyes are turned by the angle. An angle of the mouth can be obtained by, for example, calculating an angle of a line connecting the positions of the forehead and the chin tip, respectively, designated by the operator with respect to a vertical direction. The line connecting the forehead-the nose-the mouth center-the chin tip should be a straight line when a fetus is full-faced. Thus, by estimating the height of the nose, it is possible to obtain an angle of the face deviated from the front. For example, the nose is indicated by a kinked line connecting a point between eyebrows, the tip of the nose, and a point under the nose, and this line is turned by the angle of the face deviated from the front with respect to a line connecting the point between the eyebrows and the point under the nose.

Figure 3E shows an example of an image obtained as a result of the correction with respect to the angle and the turning of the part images as described above.

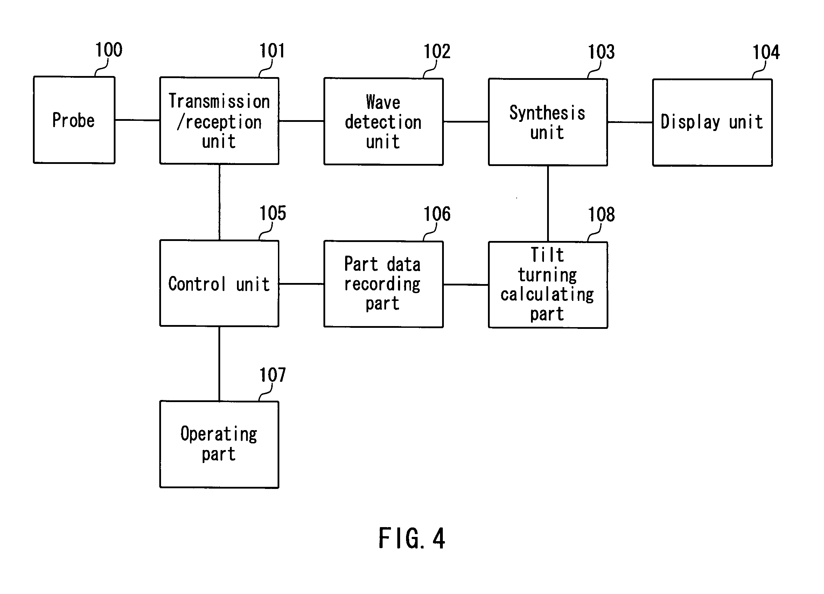

Figure 4 shows an exemplary configuration of the ultrasonic image displaying system of the present embodiment that corrects the part images as described above. In Figure 4, the configuration for transmitting/receiving ultrasonic waves and displaying an ultrasonic diagnostic image is the same as that of the first embodiment (see Figure 2), and thus the corresponding blocks are denoted with the same reference numerals and descriptions thereof will be omitted.

As is apparent from the comparison between Figure 2 and Figure 4, the ultrasonic image displaying system of the present embodiment is provided with a tilt turning calculating part 108.

In the ultrasonic image displaying system of the present embodiment, the part image data are read from the part data recording part 106 and transmitted through the tilt turning calculating part 108 to the display unit 104, where they are displayed as shown in Figure 3B. The respective part images in a state where they are read from the part data recording part 106 and displayed initially are neither tilted nor turned, which means that they are displayed as images seen from the front. When the operator selects a part image via the operating part 107, the selected part image is synthesized with the original image by the synthesis unit 103, and the synthesized image is displayed on the display unit 104. Then, if the operator designates the forehead-the nose-the mouth center-the chin tip, the tilt turning calculating part 108 calculates the tilt and the turning angle of the face, so as to correct the tilt and the turning of each of the part images for the eyes, the nose, the mouth, and the like in accordance with the calculation result, and the corrected image is displayed on the display unit 104.

### (Third Embodiment)

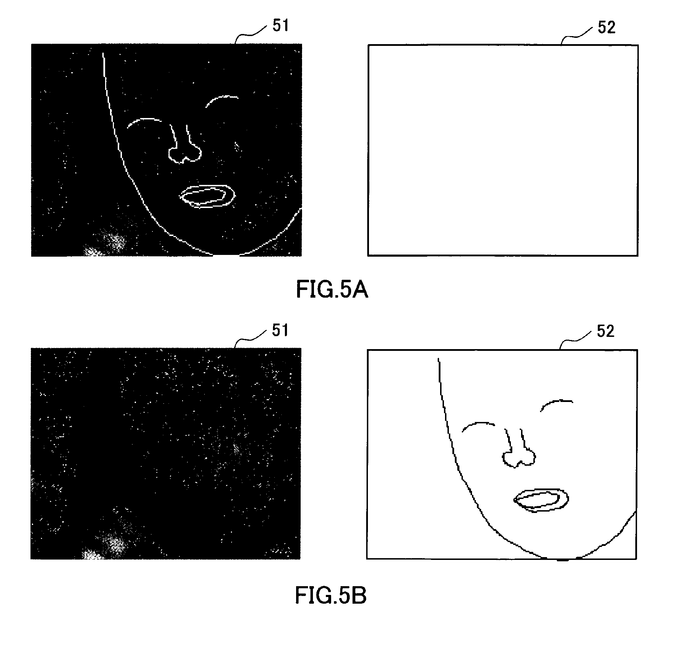

Figures 5A and 5B show an example of a manner of an image display in an ultrasonic image displaying system according to a third embodiment of the present invention.

A screen 51 shown on the left side of Figure 5A displays a state where part images are superimposed on an ultrasonic diagnostic image (original image) as described in the first and second embodiments. In the ultrasonic image displaying system of the present embodiment, another display region 52 as shown on the right side of Figure 5A is secured in addition to the screen 51. When an operator gives an instruction of movement to the ultrasonic image displaying system, the part images superimposed on the original image are extracted and moved to the secured display region 52, so that they are displayed side by side with the original image on the screen 51 as shown in Figure 5B. As a result, it is possible to produce a display that is easy for a patient to understand without spoiling the nature of the original image. Further, since the part images are created by being superimposed on the original image initially, the created part images have the correct positional relationship with the original image. When the original image and the part images are printed side by side, it is easy for the patient to provide an explanation to her family.

In the case where the part images are displayed on the different screen as shown in Figures 5A and 5B, it is preferable that a facial contour line is displayed for the sake of understandability. The facial contour line may be drawn by the operator with a pointing device or the like or drawn automatically based on shading of the ultrasonic diagnostic image, for example.

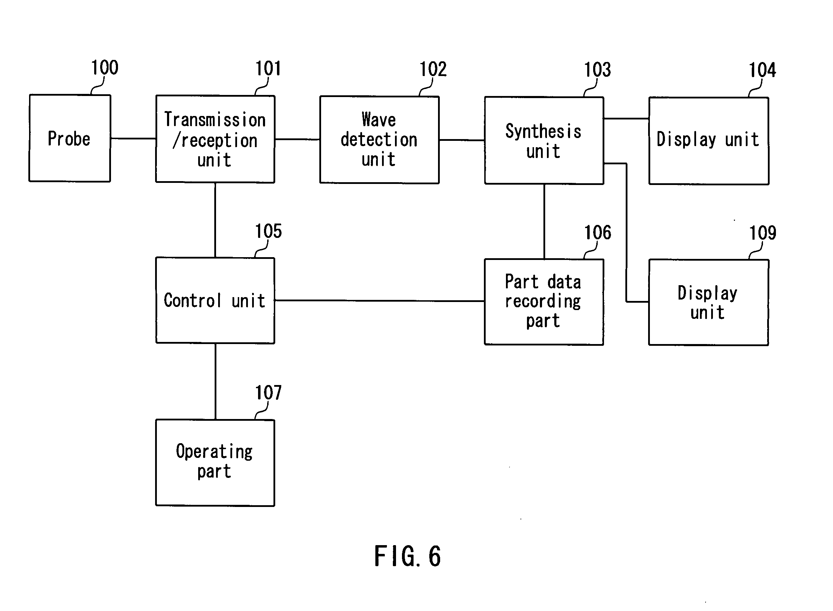

Figure 6 shows an exemplary configuration of the ultrasonic image displaying system of the present embodiment that produces the display as shown in Figures 5A and 5B.

In Figure 6, the configuration for transmitting/receiving ultrasonic waves and displaying an ultrasonic diagnostic image is the same as that of the first embodiment (see Figure 2), and thus the corresponding blocks are denoted with the same reference numerals and descriptions thereof will be omitted.

As is apparent from the comparison between Figure 2 and Figure 6, the ultrasonic image displaying system of the present embodiment is provided with a second display unit 109. When the operator superimposes the part images on the ultrasonic diagnostic image displayed on the first display unit 104 via the operating part 107, and then provides an instruction of completion of the part images to the operating part 107, the synthesis unit 103 transmits only the data of the part images to the second display unit 109 under the control of the control unit 105, while the original image is displayed on the first display unit 104. As a result, the part images are moved to the second display unit 109, and it becomes possible to compare the original ultrasonic diagnostic image and the part images visually.

In the present embodiment, the two displays are used to display the original ultrasonic diagnostic image and the part images, respectively. However, a single display may be divided into display regions, so that the ultrasonic diagnostic image and the part images are displayed side by side. This embodiment also is within the technical scope of the present invention.

In the first to third embodiments, an example was shown in which images of face parts (eyes, a nose, and a mouth) of a fetus are displayed as descriptive images. However, the part images are not limited thereto, and it is possible to display part images for any body regions such as a facial contour, a body contour, hands, and feet.

Further, the ultrasonic image displaying system according to the first to third embodiments may be provided with a configuration for printing the image obtained as a result of adding the part images to the ultrasonic diagnostic image of a fetus. This allows a patient to take home the ultrasonic diagnostic image to which the part images are added so as to show it to her family and the like. Further, in addition to the configuration for printing, the ultrasonic image displaying system may have a configuration that performs recording on a video tape or the like (analog data output) or digital data output with respect to an optical disk or the like. It is preferable that the ultrasonic image displaying system of the third embodiment outputs the original image and the part images in a form that allows easy comparison therebetween.

As described above, according to the present invention, it is possible to provide the ultrasonic image displaying system that is easy to operate and capable of providing an image that is easy to understand by superimposing the part images on the image of a fetus.

## Claims

1. An ultrasonic image displaying system, comprising:
a display unit for displaying an image of a fetus;
a part image recording unit for recording part images; and
an image synthesis unit for reading a part image corresponding to a region designated by an operator on the image displayed by the display unit from the part image recording unit, and superimposing the read part image on the image displayed by the display unit.

2. The ultrasonic image displaying system according to claim 1,
wherein the part image recording unit records as the part images a plurality of types of part images with respect to at least one facial region,
the system further comprising a selection operation unit for allowing the operator to select any of the plurality of types of part images recorded by the part image recording unit,
wherein the part image selected by the selection operation unit is superimposed on the image.

3. The ultrasonic image displaying system according to claim 1 or 2, further comprising a correction unit for correcting a turning angle of the part image in the display unit.

4. The ultrasonic image displaying system according to any one of claims 1 to 3, comprising a part image display unit for separating the plurality of part images superimposed by the image synthesis unit from the image of the fetus while preserving a relative positional relationship of the part images, and displaying the separated part images in a region different from a display region for displaying the image of the fetus.

5. The ultrasonic image displaying system according to any one of claims 1 to 3, comprising an output unit for outputting the image of the fetus and the part image added to the image in at least one form of printing, analog data output, and digital data output.

6. The ultrasonic image displaying system according to claim 4, comprising an output unit for outputting as separate images the image of the fetus and the part images displayed by the part image display unit,
wherein the output unit performs output in at least one form of printing, analog data output, and digital data output.
